Europäisches Patentamt

**European Patent Office**

Office européen des brevets

(19)

(11) Publication number: **0 261 088**
**A2**

(12)

# EUROPEAN PATENT APPLICATION

(21) Application number: **87830331.2**

(22) Date of filing: **15.09.87**

(51) Int. Cl.4: **C 08 B 37/08**
C 07 K 3/02, A 61 K 31/73,
C 07 K 15/14

(30) Priority: **19.09.86 IT 4847586**

(43) Date of publication of application:
**23.03.88 Bulletin 88/12**

(84) Designated Contracting States:
**BE DE ES FR GB GR LU NL**

(71) Applicant: **Farmaceutici Damor S.p.A.**
**27 Via S. Maria a Cubito**
**I-80145 Napoli NA (IT)**

(72) Inventor: **Riccio, Vincenzo**
**34 Via Marino Turchi**
**I-80132 Napoli NA (IT)**

(74) Representative: **Bazzichelli, Alfredo et al**
**c/o Società Italiana Brevetti S.p.A. Piazza Poli 42**
**I-00187 Roma (IT)**

(54) **Process for the production of heparanlysopeptide sulphate, the product thus obtained and its therapeutic use.**

(57) A process for the production of heparanlysopeptide sulphate, obtained from protoheparan of swine mucosa is described. Such a process comprises the purification of the proteoheparan from the other mucosa proteoglycans, the cleavage of the "protein core" from the mucopolysaccharidic component, the purification of the peptide of the proteic core showing fibrinolytic activity, the elimination of the fractions of the heparan sulphate having higher sulphatation degree and the following salification of the heparan sulphate and the fibrinolytic peptide so obtained. The use of the compound thus obtained as a fibrinolytic active agent is also described.

EP 0 261 088 A2

Bundesdruckerei Berlin

**Description**

## "PROCESS FOR THE PRODUCTION OF HEPARANLYSOPEPTIDE SULPHATE, THE PRODUCT THUS OBTAINED AND ITS THERAPEUTIC USE"

Background of the invention

Heparan sulphate is one of the glycosaminoglycuronoglycans present in animal tissues under the form of proteoglycan. The proteoglycans are made up of protein, (which constitutes the inner part, or "core protein", of the proteoglycan itself) and of a various number of side chains of glycosaminoglycuronoglycans, covalently bonded to said "core protein".

The heparan sulphate, under the form of proteoheparan, was isolated for instance from the aortic tissue (L. Jansson and U. Lindahl, Biochem. J. 1970, 117, 699) and from the liver (A. Oldberg, M. Hook, B. Obrink, H. Pertoft and K. Rubin, Biochem. J., 1977, 164, 75); it has been, moreover, demonstrated that the development of cells "in vitro" can produce heparan sulphate under the form of proteoglycan (P.M. Kraemer and D.A. Smith, Biochem. Biophys. Res. Commun. 1974, 56, 426; G.W. Conrad and G.W. Hart, Dev. Biol. 1975, 44, 253).

The structure of the proteoglycans is still widely unknown and varies considerably according to the species and tissue of origin; in general the proteoglycans have a molecular weight between 75 and 450 $\times$ 10$^3$ dalton (the protein core has a molecular weight varying from 20 to 300•10$^3$ dalton).

The pharmacological properties of these substances are similarly not well known; the antithrombogenic properties of the luminal surface of the blood vessels are in general attibuted to these substances (J.E. Kirk, Nature 1959, 184, 369; J. Gore and B.J. Larkey, J. Lab. Clin. Med., 1960, 56, 839).

The mechanism of this functional property seems to be the fact that the deposit proteoglycans of the surface of the endothelial cells are degraded by heparinase (V. Buonassini and M. Rott, Biochem. Biophys, Acta, 1975, 385, 1; M. Simionescu, N. Simionescu, J.E. Silbert and G.E. Palade, J. Cell. Biol., 1981, 90, 614) and by other glycosaminoglycan-specific enzymes; in this way the respective mucopolysaccharides are liberated; said mucopolysaccharides could interact with the thrombin, with the antithrombin III (A. Teien, U. Abilgard and M. Hook, Tromb. Res., 1976, 8, 859; M.V.C. Hatton, L.R. Berry and E. Regoeczi, Thromb. Res., 1978, 13, 655) and with all the substances which play a role in the blood vessel coagulative homeostasis.

Recently a proteoglycanic fraction extracted from the blood vessels of the calf has been described and said fraction was produced only by the endothelium and showed an anticoagulant activity (J.A. Marcum, L. Fritze, S.J. Galli, G. Karp and R.D. Rosemberg, Am. J. Physiol., 1983, 245, H 725).

The lack of purification methodologies by which unaltered products in sufficiently high quantities can be obtained, is one of the main grounds for the lack of knowledge concerning the structure and functional properties of the proteoglycans. As a matter of fact the processes of industrial production of the glycosamino-glycuronoglycans, belonging to the state of the art, provide for the proteolytic digestion of the used tissue. Such treatment has the main result of the cleavage of the glycosaminoglycuroglycans from the core protein, which is in part degraded. The digested product is made up of a mixture of oligopeptides and glycosaminoglycuronoglycans and of course other tissue components, such as lipids, nucleic acids etc.

From the foregoing it follows that the polysaccharide component of the proteoglycan has been mainly used up to now; the research of the properties of the protein core up to now has been neglected.

State of the art

Heparin and heparan-sulphate are shown to be the most studied and the most interesting among the glycosaminoglyconoglycans in therapeutical use.

In particular the pharmacological profile of the heparan sulphate, the proteoglycan of which is the base of the present invention, has been widely demonstrated; said profile has been characterized by anticoagulant, fibrinolitic, antilipemic and antiinflamatory properties. These properties are strongly influenced by the species and by the origin tissue of the heparan sulphate and by the different processes of purification. This is in agreement with the heterogeneity of this class of macromolecules and with a partial degradation which they undergo as a consequence of chemical and/or enzymatic treatment during the purification processes.

As a matter of fact various industrial processes for the purification of heparan sulphate, in a substantially pure form, have recently been developed. These processes employ various starting materials such as the "by-products" of the manufacture of heparins starting from swine mucosa and proteoglycans of swine aorta and comprise various degrees of purification from a heparan sulphate purified by the other mucopolysaccharide families to a heparan sulphate fractioned according to the molecular weight. In all these cases heparan sulphates showing the pharmacological profile typical of this family of substances have been obtained; such a profile has been more or less influenced by the degree of purity and by the type of fractioning. Heparan sulphates showing different pharmacological profiles according to the administration route (per os or by parenteral administration) heparans showing low anticoagulant activity etc. have been described.

None of these processes comprises the recovery of the "core protein" of the proteoglycan and the study of the properties and the interactions of the same with the polysaccharidic mojety.

## Summary of the invention

An object of the present invention is a process for the production of heparanlysopeptide sulphate from swine mucosa, comprising the following steps:

a) grinding the swine mucosa;

b) delipidating with acid the ground mucosa;

c) extracting the proteoglycans by a solubilizing agent up to solubilizing the macromolecular components of the delipidated mucosa;

d) purifying the proteoglycans from said macromolecular components on a column containing hydrofil type anionic exchangers;

e) separating the proteoglycans from the nucleic acids remaining after purification;

f) separating the proteoheparan from the other proteoglycans having molecular weight higher than 100-200 × $10^3$ dalton by gel filtration;

g) cleavage of the protein core from the proteoheparan by means of treatment with sodium hydroxide and sodium boronhydride;

h) separating the protein core from the proteoheparan on cation exchanger containing column;

i) separating the heparan sulphate from the proteoheparan on a column containing anion exchanger;

j) subjecting to a pancreatinic lysis the core protein to obtain a proteic lysate;

k) eliminating the high molecular weight proteins from the proteic lysate by selective precipitation;

l) precipitating the polypeptides from the proteic lysate with ammonium sulphate and dissolving in water solution said precipitated polypeptides;

m) purifying the lysopeptide having molecular weight of 12-14 × $10^3$ dalton from said water solution by gel chromatography;

n) eliminating the high sulphatated fraction of the heparan sulphate obtained in step i), and

o) salifying the heparan sulphate and the lysopeptide to obtain heparanlysopeptide sulphate.

The heparanlysopeptide sulphate, obtained by the process of the present invention, the heparan sulphate obtained, in a substantially pure form, after the step n) of the process of the present invention and the lysopeptide obtained after the step m) of the process of the present invention can be considered, in view of their properties and characteristics, as new substances, and are further objects of the present invention. The above mentioned substances, according to an additional aspect of the present invention, are new fibrinolytic, antithrombotic and normocoagulative agents. Said agents can be formulated into pharmaceutical compositions comprising a therapeutically effective amount for promoting fibrinolysis of the heparanlysopeptide sulphate, of the heparan sulphate and of the lysopeptide obtained with the process of the present invention and a pharmaceutically acceptable vehicle.

The peptide isolated by the "core protein" strengthens moreover the fibrinolytic properties of the heparan sulphate obtained according to the process of the present invention.

## Example of preparation of the heparanlysopeptide sulphate

10 kg of tissue (swine mucosa) are ground at low temperature, then they are delipidated by agitation with 3 volumes of acetone. The extraction is repeated until complete delipidation is obtained. The ground and delipidated organ is dissolved in one volume of water.

Guanidine chloride up to a concentration of about 4 M and phosphate buffer up to the concentration of about mM (pH 6-7) are added to the suspension; the guanidine chloride extracts (or solubilizes) the macromolecular components of the tissue (proteoglycans included).

The suspension is kept at 4°C for 24 hours under mild agitation. After the extraction phase, the suspension is filtered in a filter press.

The limpid filtrate is then charged on a 30 cm diameter by 70 cm height column containing hydrofil type anion exchangers; thus the polianions, that is proteoglycans and nucleic acids, are bonded to the column by this process.

After this application the column is washed with phosphate buffer 50 mM, pH 6-7, in sodium chloride 0,5 M (all the proteins and a part of the nucleic acids are eliminated); then the column is discharged by elution with sodium chloride 2 M (the proteoglycans and the residual nucleic acids are eluted from the column). The eluate is concentrated up to 1/10 of its volume and precipitated with two volumes of ethanol.

The precipitate is dissolved in a phosphate buffer and the residual nucleic acids are eliminated according to one of the following alternative methods:

a) by action of endonuclease (RNA-ase; DNA-ase);

b) by precipitation of the nucleic acids at pH 1,5 using HCl 1N;

c) by new column application on anion exchangers, said column being equilibrated with sodium acetate at pH 6, and following washing with sodium chloride 0,8 M and sodium acetate 50 mM.

The precipitate is dissolved in two volumes of:
- phosphate buffer pH 7, in case a);
- water in case b).

In case c) the column is eluted with a 2 M solution of NaCl.

Yield in proteoglycans: about 20-30 g.

The proteoglycan solution (after concentration in the case c)) is gel filtered on Sepharose CL-6B in order to separate the proteoheparan from the other proteoglycans (lower molecular weight about 100-200 × $10^3$

dalton).

Yield in proteoheparan; 10-15 g (as a solution).

Sodium hydroxide q.b. to 50 mM and sodium boronhydride q.b. to 0,2 M are added to the proteoheparan solution obtained (the sodium hydroxide cleaves the protein from the mucopolysaccharide, the boronhydride protects it from degradation).

The solution is maintained at a temperature of 40°C for 24 hours, then it is neutralized with acetic acid 1 M. The solution is then charged on a column which contains a cation exchanger, equilibrated with a phosphate buffer 50 mM at pH 5.

The column is then washed with the same buffer and discharged with sodium chloride 0,5 M.

The eluate is then concentrated by ultrafiltration; the concentrate is then liophylized (protein core) and used for the purification of the fibrinolitic lysopeptide (LPF concentrate). Yield: about 4-8 g.

The washing liquid of the column is charged on a column of 70 × 10 cm containing an anion exchanger, at pH 7. The column is then washed with sodium chloride 0.5 and discharged with sodium chloride 2 M. The eluate is concentrated by reverse osmosis up to 1/10 of its initial volume and precipitated with 2.5 volumes of ethanol. The precipitate is then dissolved again in water and precipitated again with 4 volumes of ethanol. The precipitate is then washed twice with acetone and dried under vacuum (HS concentrate): yield: 5-10 g.

## Purification of the fibrinolytic lysopeptide

The concentrate (LPF) is suspended again in water under agitation up to complete mixing; the pH is then raised up to 7 by addition of a phosphate buffer. The suspension is heated at 45°C, then, under continuous agitation, pancreatin (50 mg/5 g) in 2% calcium chloride water solution is added.

The heating and mild agitation are maintained for ten hours with a continuous control of the pH. The mixture is then cooled at 20 C and the pH brought down to 2.5 by successive additions of HCl 1N (elimination of the high molecular weight proteins). The mixture is kept under agitation for three hours and then it is centrifugated at 5000 rpm. The residue is collected using an acid solution and then, after agitation, again centrifugated. The collected supernatants are filtered to complete limpidness, then they are brought to pH 6 by addition of sodium hydrate.

Ammonium sulphate, under continuous agitation, up to the concentration of 40%, is then added to the solution. The solution is kept under agitation for 30 minutes then, after a 24 hour rest, a flocculate is formed on the bottom. This flocculate is then dissolved in a water solution containing 0.5 M of sodium chloride and filtered on a 0.45 mm filter.

The solution is charged in a 20 cm × 10 cm column of Sephadex G 75 eluting with phosphate buffer pH 7,8.

The lysopeptide in object is the eluted fraction having a molecular weight of 12-14 × $10^3$ dalton. The lysopeptidic fraction thus obtained is then liophylized.

Yield in lysopeptide: 16-32 mg.

## Purification of the heparan sulphate

The heparan sulphate obtained according to the above described process (HS concentrate) in contaminated by highly coagulative fractions. This could provoke a different profile of pharmaceutical action according to the absorbtion route (the high sulphatated fractions show an anticoaguative activity); this problem is solved by elimination of said fractions by:

a) solubilization of the HS concentrate in calcium acetate 5% and acetic acid 0.5 M; or

b) precipitation by adding ethanol under agitation up to 20-22%.

Yield in heparan sulphate: 4-8 g.

## Salification of the heparanlysopeptide sulphate

The lysopeptide obtained shows an isoelectric point at a basic pH. This makes its salification with polianions possible, as for example the glycosaminoglycuronoglycans. The salification is carried out by precipitation with solvent or by liophylization after purification by ultrafiltration. In this way the heparanlysopeptide sulphate is obtained.

## Chemical-analytical characteristics of the heparanlysopeptide sulphate

The characterization of the components of the heparanlysopeptide sulphate product (heparan sulphate and fibrinolytic lysopeptide) is carried out as follows:

500 mg of the compound are dissolved in 20 ml of phosphate buffer at pH 7 and charged on a column (2.5 cm ∅ 50 cm h) packed with Sephadex CM 50, equilibrated with the same buffer. The column is washed with buffer up to the elution of the heparan sulphate. A solution of NaCl 0.6 M in phosphate buffer at pH 7 is added; the elution of the peptide is continuously followed spectrophotometrically at 280 nm. The fractions corresponding to the absorbtion peak are collected and concentrated by ultrafiltration at 4°C.

## Chemical-analytical characteristics of the heparansulphate

Molecular weight: 500 - 30,000 dalton; determined on a column of 100 cm height × 1 cm diameter of Sephacryl S 200 superfine, equilibrated in guanidine chloride 2 M. The elution is carried out with the same solution of guanidine chloride with a flow of 7 ml/hour; the collected fractions are then diluted 1:5 with $H_2O$ and in portions of 100 mole each the uronic acids were determined using the orcinol method.

The points thus obtained are interpolated and by the elution average volume of each peak the Kav (elution coefficient) is determined, which was compared with a calibration line (Kav/log MW) obtained using dextrans having an exactly defined molecular weight.

- Average sulphatation degree: $1.0 \pm 1.3$ $SO_4$/esosamine (determined by gravimetric method).
- Hydruration degree: 30-35% (calculated by the carbazol/orcinol relation).
- Electrophoresis carried out according to R. Cappelletti et al (Anal. Biochem., 1979, 99, 311).

The electrophoresis data are summarized in the following Table 1.

### Table 1

| Peak N° | Position | Intensity 1000 | Intensity % |
|---------|----------|----------------|-------------|
| 1 | 24,9 | 20,6 | 0,8 |
| 2 | 34,5 | 0,2 | 0,0 |
| 3 | 40,8 | 2554,7 | 99,2 |

Total intensity: 2575,6 = 100,0%.

### Chemical-analytical characteristics of fibrinolytic lysopeptide

- Molecular weight: the molecular weight of the lysopeptide is $12\text{-}14 \times 10^3$ dalton. The molecular weight was determined by gel filtration on a column of Sephadex G.100 (diameter $2.5 \times 100$ cm height) calibrated with Blue dextran (Vo) and $H_2CrO_4$ (VE). Cythocrome C, ovalbumin, bovine serum albumin and tranferin have been used as reference substances.

- Electrophoresis: method on polyacrylamide gel.

The gel with sodium dodecilsulphate and the buffers were prepared according to Weber and Osborn (J. Biol. Chem. 1969, 244, 4406).

The sample solution is incubated at 37°C for 2 hours in phosphate buffer 0.01 M at pH 7 with sodium dodecil sulphate at 1%; Comassie Brillant Blue 0.25% in methanol at 50% and pure acetic acid (10:1) are used as dying solution. The electrophoretic data are reported in Table 1A.

### Table 1A

| Peak N° | Position | Intensity 1000 | Intensity % |
|---------|----------|----------------|-------------|
| 1 | 46,5 | 2651,5 | 100,0 |

Total intensity: 2651,5 = 100,0%.

### Experimental pharmacological portion

The pharmacological profile of the heparanlysopeptide sulphate, obtained according to the process of the present invention, has been evaluated both "in vitro" and "in vivo" on voluntary patients of both sexes and aging between 45 and 70 years suffering from atheovenous pathologies.

In order to carry out the "in vitro" tests, the samples were dissolved in physiological saline solution and brought to the final concentration of 1 mg/ml.

The determinations were carried out twice together with the control, and with a control to which physiologic saline solution in a quantity (microliters) equal to the added sample was added.

On a human plasma substrate of voluntary patients the prothrombine time (Hepato-Quick) the partial activated thromboplastin time (PTTa), the thrombin time (TT) and the polimerizable fibrinogen as an indication of the interaction with the coagulative process were evaluated.

The influence on the fibrinolytic process was evaluated carrying out determinations of the plasminogen, of the primary inhibitor alfa-2-antiplasmina and, as a general indication, the time of lysis of the euglobulins ELT.

The influence on the concentrations of antithrombin III (AT III) have also been studied.

The results are shown in the following Table II.

## Table II

| Test | Control | Control + physiologic saline solution | Control + heparan-lysopeptide sulphate 1 mg/ml |
|---|---|---|---|
| Hepato Quick % | 94 | 94 | 98 |
| TTPa sec. | 26,5 | 24,5 | 96 |
| TT sec. | 16 | 17 | not coagulable |
| Fibrinogen % | 340 | 335 | 300 |
| Alfa-2-antiplasmin (IU/ml) | 0,776 | 0,758 | 0,521 |
| Plasminogen mg % | 12,8 | 12,8 | 11,4 |
| AT III (IU/ml) | 10,5 | 10,3 | 11,39 |
| ELT min. | 228 215 | 145 | |

As to the "in vitro" action on the fibrinolytic process, in a second set of tests the influence of the fibrinolytic component of the salt (lysopeptide) on the fibrinolytic activity of the heparan sulphate has been evaluated.

To this purpose the components of the heparanlysopeptide sulphate were fractioned by chromatography on anionic and cationic exchangers, according to the process above referred to, and the single compounds thus obtained were dissolved in solution up to the final concentration of 1 mg/ml. The fibrinolytic activity was tested with the test of the time of lysis of the euglobulins in the same experimental conditions hereinabove referred to.

The results are as follows:

## Table III

| Compound    1 mg/ml | ELT min. |
|---|---|
| Control | 228 |
| Control plus physiological saline solution | 215 |
| Heparan sulphate | 166 |
| Heparanlysopeptide sulphate | 145 |

From the reading of the table, it follows that the lysopeptide can strengthen the fibrinolytic activity of the heparan sulphate; this can be explained as the result of a complementarity of action in the various steps of the same fibrinolytic process. In fact, the lysopeptide, if applied on a layer of fibrin, in absence of plasminogen, said layer of fibrin being prepared according to T. Astrup and S. Mullertz (Arch. Biochem., 1952, 40, 346) provokes at concentrations of 1-3 /ml (0,01 ml applied) a marked halo of lysis in the fibrin; this demonstrates a plasminic type activity. Moreover, it has been widely shown in literature that the fibrinolytic effect of the heparan sulphate can be explained as the result of a complex mechanism comprising an action on the structure of the fibrin, a more efficient use of the plasminogen to activate the activators, an interaction with specific factors as, for instance, the thrombin, but it cannot be in any way explained on the basis of a plasminic type effect.

In vivo the compoud was administered both orally and parenterally, in a single administration; the interactions with the coagulative and the fibrinolytic processes were evaluated at different times.

In view of the fact that the product shows the same qualitative profile of action both for the oral administration and for the parenteral administration, and that the amount of the effects is dose dependent, in the following the data obtained for the minimum tested doses are listed; said minimum dose is the best indication for a thorough evaluation of the interaction with the coagulative and fibrinolytic processes.

### Table IV

Effect of heparanlysopeptide sulphate on the Hepato Quick time (T-EQ), on the partial activated thromboplastin time (PTTa) and on the thrombin time (TT).

| Administration route | Dose mg | Maximum variation % with respect to the basal | | |
|---|---|---|---|---|
| | | T-EQ | PTTa | TT |
| os | 20 | +0,3% (4) | +4,0 (4) | +2,0 (4) |
| i.m. | 20 | +1,4 (2) | +6,5 (2) | +4,5 (2) |

The figures in brackets indicate the time in hours of maximum activity from the administration.

## Table V

Effect of heparanlysopeptide sulphate on the Xa factor
and on Antithrombin III (AT III)

| Administration route | Dose mg | Maximum variation % with respect to the basal | |
| --- | --- | --- | --- |
| | | Xa | AT III |
| os | 20 | − 7,2 (6) | + 4,5 (6) |
| i.m. | 20 | −15,5 (4) | + 6,8 (4) |

The figures in brackets indicate the time in hours of
maximum activity from the administration.

## Table VI

Effect of heparanlysopeptide sulphate on the plasmatic
rates of fibrinogen (FB), of alfa-2-antiplasmin ($\alpha$-2 AP)
and of plasminogen (PA)

| Administration route | Dose mg | Maximum variation % with respect to the basal | | |
| --- | --- | --- | --- | --- |
| | | FB | $\alpha_2$AP | PA |
| os | 20 | − 7,4 (6) | −12,5 (6) | −10,8 (6) |
| i.m. | 20 | −12,5 (4) | −18,0 (4) | −16,4 (4) |

The figures in brackets indicate the time in hours of
maximum activity from the administration.

## Table VII

**Effect of heparanlysopeptide sulphate on the time of lysis of the euglobulins (ELT) and on the time of lysis of the euglobulins after venous stasis (ELT SV).**

| Administration route | Dose mg | Maximum variation % with respect to the basal | |
|---|---|---|---|
| | | ELT | ELT SV |
| o s | 20 | -33,0 (6) | -28,2 (6) |
| i.m. | 20 | -40,7 (4-6) | -37,5 (4-6) |

The figures in brackets indicate the time in hours of maximum activity from the administration.

The above referred data show that the heparanlysopeptide sulphate, obtained according to the process of the present invention, has the following pharmacodynamical properties:

a) a significant variation of the parameters of the coagulative process can be determined neither in the case of oral administration, nor in the case of parenteral administration (poor anticoagulative activity);

b) the fibrinolytic effect is remarkable in both the administration routes; this can be shown both by studying the influence on the global process, in normal conditions and after venous stasis, and by studying the influence on the various steps of the same process;

c) the evaluation of the fibrinolytic effect in vitro shows that the lysopeptide can strengthen the fibrinolytic effect of the heparan sulphate.

In conclusion it can be shown that the heparanlysopeptide sulphate has marked fibrinolytic properties. As a matter of fact, preliminary tests in repeated treatment activated in an oral administration in doses comprised between 40 and 320 mg/die, also in gastroresistent form, and in a parenteral route of administration (intramuscular and intravenous), in the case of doses between 20 and 200 mg/die, showed significant results in the profilaxis and therapy of all the atherovenous pathological forms (thrombosis, thromboembolism, etc.); during these administrations no side effect could be determined.

## Claims

1. A process for the production of heparanlysopeptide sulphate from swine mucosa, comprising the following steps:

a) grinding the swine mucosa;

b) delipidating with acid the ground mucosa;

c) extracting the proteoglycans by a solubilizing agent up to solubilizing the macromolecular components of the delipidated mucosa;

d) purifying the proteoglycans from said macromolecular components on a column containing hydrofil type anionic exchangers;

e) separating the proteoglycans from the nucleic acids remaining after purification;

f) separating the proteoheparan from the other proteoglycans having molecular weight higher than 100-200 $\times$ $10^3$ dalton by gel filtration;

g) cleaving the protein core from the proteoheparan by means of treatment with sodium hydroxide and sodium boronhydride;

h) separating the protein core from the proteoheparan on a cation exchanger containing column;

i) separating the heparan sulphate from the proteoheparan on a column containing anion exchanger;

    j) subjecting the core protein to a pancreatininc lysis to obtain a proteic lysate;

    k) eliminating the high molecular weight proteins from the proteic lysate by selective precipitation;

    l) precipitating the polypeptides from the proteic lysate with ammonium sulphate and dissolving in water solution said precipitated polypeptides;

    m) purifying the lysopeptide having molecular weight of 12-14 $\times$ $10^3$ dalton from said water solution by gel chromatography;

    n) eliminating the high sulphatated fraction of the heparan sulphate obtained in step i), and

    o) salifying the heparan sulphate and the lysopeptide to obtain heparanlysopeptide sulphate.

2. The process according to claim 1, in which the extraction of the proteoglycans (step c)) is carried out by adding guanidine chloride up to the concentration 4 M and phosphate buffer up to the concentration 50 mM (pH 6-7).

3. The process according to claim 1, in which the purification of the proteoheparan from the other proteoglycans (step f)) is carried out by gel filtration on Sepharose CL-6B.

4. The process according to claim 1, in which the cleavage of the protein from the proteoheparan (step g)) is carried out by adding sodium hydroxide up to the concentration of 50 mM and sodium boronhydride up to the concentration of 0,2 M.

5. The process according to claim 1, in which a peptide, having fibrinolytic activity, is obtained from the protein of the proteoheparan by means of pancreatinic lysis, elimination of the high molecular weight protein by addition of HCl 1N and following purification by gel chromatography on Sephadex G 75 eluting with phosphate buffer pH 7-8.

6. The process according to claim 1, in which the elimination of the high sulphatated fractions of the heparan sulphate (step n)) is carried out by solubilization of the heparan sulphate concentrate in 5% calcium acetate plus ascetic acid 0,5 M and following addition of ethanol under agitation up to 20-22%.

7. Heparanlysopeptide sulphate, substantially pure, obtained from step o) of the process as claimed in claim 1.

8. Heparan sulphate, substantially pure, having molecular weight from 5 to 30 $\times$ $10^3$ dalton, average sulphation degree 1,0-1,3 $SO_4$ groups/esosamine, hydride degree 30-35%, obtained from step n) of the process as claimed in claim 1.

9. Lysopeptide having molecular weight of 12-14 $\times$ $10^3$ dalton and the following electroforetic charateristics: peak No 1, position 46,5, intensity (1000) 2651,5, intensity (%) 100,0, following the polyacrylamide gel method and the method of Weber and Osborn.

10. Use of the heparanlysopeptide sulphate as claimed in claim 7, for the manufacture of a medicament having fibrinolytic activity.

11. Use of the heparan sulphate as claimed in claim 8 for the manufacture of a medicament having fibrinolytic activity.

12. Use of the lysopeptide as claimed in claim 9 for the manufacture of a medicament having fibrinolytic activity with prevailing plasminic activity.

13. A pharmaceutical composition having fibrinolytic activity, comprising the heparanlysopeptide sulphate as claimed in claim 7 and a pharmaceutically acceptable vehicle.

14. A pharmaceutical composition having fibrinolytic activity, comprising the heparan sulphate as claimed in claim 8 and a pharmaceutically acceptable vehicle.

15. A pharmaceutical composition having fibrinolytic activity with prevailing plasminic activity, comprising the lysopeptide as claimed in claim 9 and a pharmaceutically acceptable vehicle.